# EUROPEAN PATENT APPLICATION

(11) **EP 1 946 758 A1**
(43) Date of publication of application: **23.07.2008**
(21) Application number: 07001081.4
(22) Date of filing: 18.01.2007
(51) Int. Cl.: A61K 31/365, A61P 35/02

(54) **Treatment of acute myeloid leukemia**

(71) Applicant: PIERRE FABRE MEDICAMENT, Castres Cedex 81106 (FR)
(72) Inventor: Brandely-Talbot, Maud, 75017 Paris (FR); Hurteloup, Pierre Patrick, 75017 Paris (FR)
(74) Representative: Ahner, Francis

(57) **Abstract**

The invention is directed to the use of an ester derivatives of triptolide, tripdiolide, 16-hydroxytriptolide, a pharmaceutically acceptable salt thereof, or a combination thereof, for the manufacture of a medicament comprising the triptolide prodrug for the treatment of acute myeloid leukemia.

## Description

### BACKGROUND

### Field of the Invention

The invention taught herein is generally directed to the treatment of acute myeloid leukemia using ester derivatives of triptolide, tripdiolide or 16-hydroxytriptolide.

### Description of Related Art

Leukemia encompass a wide variety of blood cancers corresponding to different pathological profiles, population, evolution and treatments. Adult acute myeloid leukemia (AML) is a type of cancer in which the bone marrow makes abnormal myeloblasts (a type of white blood cell), red blood cells, or platelets. AML is a cancer of the blood and bone marrow that usually gets worse quickly if left untreated. It is the most common type of acute leukemia in adults and is also called acute myelogenous leukemia, acute myeloblastic leukemia, acute granulocytic leukemia, and acute nonlymphocytic leukemia.

In AML, the stem cells usually develop into a type of immature white blood cell called myeloblasts (or myeloid blasts). The myeloblasts are abnormal and do not mature into healthy white blood cells and, sometimes, too many stem cells develop into abnormal red blood cells or platelets. These abnormal white blood cells, red blood cells, or platelets are also called leukemia cells or blasts. Leukemia cells are unable to do their usual work and can build up in the bone marrow and blood resulting in less room for healthy white blood cells, red blood cells, and platelets, creating infection, anaemia, or easy, uncontrollable bleeding. The leukemia cells can spread outside the blood to other parts of the body, including the central nervous system, skin, and gums.

A number of compounds from the Chinese medicinal plant Tripterygium wilfordii have been identified as having immunosuppressive and/or anticancer activity. Representative compounds which have been isolated from TW include triptolide, 16-hydroxytriptolide, triptophenolide, tripdiolide, and celastrol, but the administration and therapeutic effectiveness of these compounds has been limited by their low water solubility. As a result, chemical derivatives and/or prodrugs of such natural products have been sought to overcome the solubility drawbacks and increase their therapeutic efficacy. Such pursuits include those taught in U.S. Patent Nos. 5,663,335; 6,150,539; 6,548,537; and U.S. Published Application No. 20040235943. It is an object of the present invention to provide an improved method of treating acute myeloid leukemia.

### SUMMARY

The invention taught herein is generally directed to the treatment of acute myeloid leukemia using ester derivatives of triptolide, tripdiolide or 16-hydroxytriptolide. In some embodiments, the invention includes the use of a triptolide prodrug, or a pharmaceutically acceptable salt thereof, having the following structure: 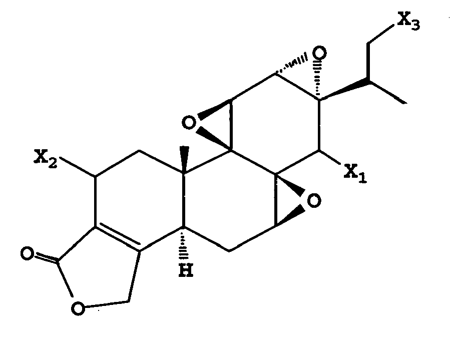 where X¹ is OH or OR¹, and X² and X³ are independently OH, OR¹ or H, with the proviso that at least one of X¹, X² and X³ is OR¹, and at least one of X² and X³ is H; and R¹ is -C(O)-Y-Z. Preferably X₁ is OH or OR₁ and X₂ and X₃ are H. Y can be a branched or unbranched C₁-C₆ alkyl or alkenyl chain, preferably C₂ alkyl chain. Z can be COOR², NR³R^{3'}, or ⁺NR⁴R^{4'}R^{4"}, where R² is a cation (e.g., in one embodiment R² is a metal ion, such as an alkali or alkali earth ion, preferably Na⁺ or K⁺. In an alternative embodiment, R² is an organic ammonium ion, protonated if necessary, preferably selected from lysine, triethylamine, or tris(hydroxymethyl)aminomethane. Preferably, R² is Na⁺, tris(hydroxymethyl)aminomethane or lysine); R³ and R^{3'} can be independently H or branched or unbranched C₁-C₆ alkyl, hydroxyalkyl, or alkoxyalkyl, or R³ and R^{3'} taken together can form a 5- to 7-member heterocyclic ring whose ring atoms can be selected from the group consisting of carbon, nitrogen, oxygen and sulfur. The ring atoms can include 2 to 6 carbon atoms and one or more nitrogen atoms, and optionally one or more oxygen or sulfur atoms, and the ring can be unsubstituted or substituted with one or more groups selected from R⁵, OR⁵, NR⁵R⁶, SR⁵, NO₂, CN, C(O)R⁵, C(O)NR⁵R⁶, OC(O)R⁵, OC(O)NR⁵R⁶, and halogen, where R⁵ and R⁶ can be independently hydrogen, lower alkyl or lower alkenyl; and R⁴, R^{4'}, and R^{4"} can be independently branched or unbranched C₁-C₆ alkyl, hydroxyalkyl, or alkoxyalkyl groups. The triptolide prodrug, or a pharmaceutically acceptable salt thereof can be used for the manufacture of a medicament, optionally in a pharmaceutically acceptable vehicle, for the treatment of acute myeloid leukemia.

In some embodiments, the triptolide prodrug, or a pharmaceutically acceptable salt thereof, has the following structure: 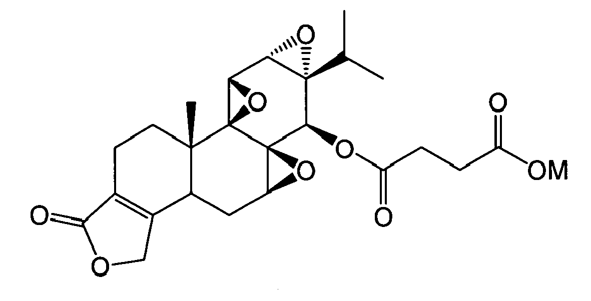 M can be H or a cation.

In many embodiments, the cation can be selected from a group consisting of a metal or an organic amine. In these embodiments, the metal can be Na or K, and the organic amine can be lysine, triethylamine, or tris(hydroxymethyl) aminomethane.

In some embodiments, the triptolide prodrug is administered in an amount ranging from 5.0 mg/m²/day to 9.0 mg/m²/day, from 5.2 mg/m²/day to 8.7 mg/m²/day, from 5.7 mg/m²/day to 8.5 mg/m²/day, or in an amount of about 7.0 mg/m²/day, or any range therein.

In some embodiments, the acute myeloid leukemia can be refractory or relapsed, and the treatments can result in complete remission with pancytopenia, complete remission without full platelets recovery, or complete remission.

### BRIEF DESCRIPTION OF THE FIGURES

FIG. 1 shows a scheme for preparing a carboxylated triptolide compound in accordance with the invention;
FIG. 2 shows a scheme for preparing amino derivatives of triptolide in accordance with the invention;
FIG. 3 shows a scheme for preparing mono- and diaminoester derivatives of 16-hydroxytrlptolide; and,
FIG. 4 shows a scheme for preparing a 14-aminoester derivative of 16-hydroxytriptolide by means of protection and deprotection of the 16-hydroxyl group.

### DETAILED DESCRIPTION OF THE INVENTION

### I. Definitions

The terms below have the following meanings unless indicated otherwise.

"Triptolide derivatives" or "triptolide analogs" refers to derivatives of triptolide, 16-hydroxytriptolide and tripdiolide (2-hydroxytriptolide) which are derivatized at one or more hydroxyl groups as described above.

"Alkyl" refers to a fully saturated monovalent or divalent radical containing carbon and hydrogen, and which may be cyclic, branched or a straight (unbranched) chain. Examples of alkyl groups are methyl, ethyl, n-butyl, n-heptyl, isopropyl, 2-methylpropyl, cyclopropyl, cyclopropylmethyl, cyclobutyl, cyclopentyl, cyclopentylethyl, and cyclohexyl.

"Lower alkyl" refers to an alkyl radical of one to four carbon atoms, as exemplified by methyl, ethyl, n-propyl, i-propyl, n-butyl, i-butyl, and t-butyl.

"Alkenyl" refers to a monovalent or divalent unsaturated, preferably mono-unsaturated, radical containing carbon and hydrogen, and which may be cyclic, branched or a straight chain. "Lower alkenyl" refers to an alkenyl radical of one to four carbon atoms.

"Heteroatom" refers to carbon, nitrogen, oxygen, or sulfur.

"A 5- to 7-member heteroatom ring containing 2 to 6 carbon atoms" refers to a heterocyclic ring whose chain atoms include one or more nitrogen atoms and, optionally, one or more oxygen or sulfur atoms. Examples are piperidine, piperazine, morpholine, and pyrrolidine.

"Alkoxyalkyl" refers to an alkyl group as defined above, additionally containing an alkoxy substituent. Preferably, the alkyl portion of the alkyloxy substituent is a lower alkyl group.

A "heterocycle" refers to a non-aromatic ring, preferably a 5-to 7-membered ring, whose ring atoms are selected from the group consisting of carbon, nitrogen, oxygen and sulfur. Preferably, the ring atoms include 3 to 6 carbon atoms. Such heterocycles include, for example, pyrrolidine, piperidine, piperazine, and morpholine.

The term "pharmaceutically acceptable salt" encompasses carboxylate salts having organic and inorganic cations, such as alkali and alkaline earth metal cations (for example, lithium, sodium, potassium, magnesium, barium and calcium); ammonium; or organic cations, for example, dibenzylammonium, benzylammonium, 2-hydroxyethyl ammonium, bis(2-hydroxyethyl)ammonium, phenylethylbenzylammonium, and the like. Other cations encompassed by the above term include the protonated form of procaine, quinine and N-methylglucosamine, and the protonated forms of basic amino acids such as glycine, ornithine, histidine, phenylglycine, lysine, and arginine.

The term also includes salts formed by standard acid-base reactions with basic groups, such as amino groups, having a counterion derived from an organic or inorganic acid.

Such acids include hydrochloric, sulfuric, phosphoric, acetic, succinic, citric, lactic, maleic, fumaric, palmitic, cholic, pamoic, mucic, D-glutamic, D-camphoric, glutaric, phthalic, tartaric, lauric, stearic, salicylic, methanesulfonic, benzenesulfonic, sorbic, picric, benzoic, cinnamic, and the like.

For the purpose of the current disclosure, the following numbering scheme is used for triptolide and triptolide analogs:

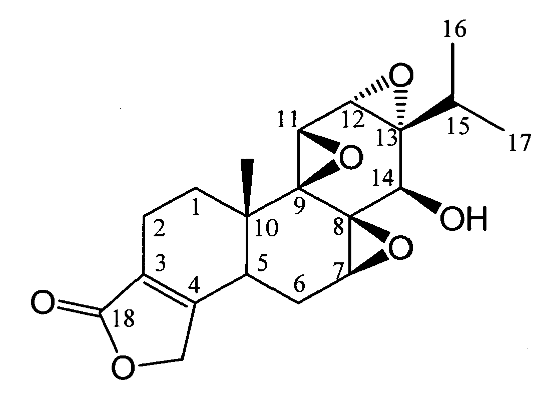

In some embodiments, the invention is directed to the treatment of acute myeloid leukemia using ester derivatives of triptolide, tripdiolide or 16-hydroxytriptolide. In some embodiments, the invention includes the use of a triptolide prodrug, or a pharmaceutically acceptable salt thereof, having the following structure: 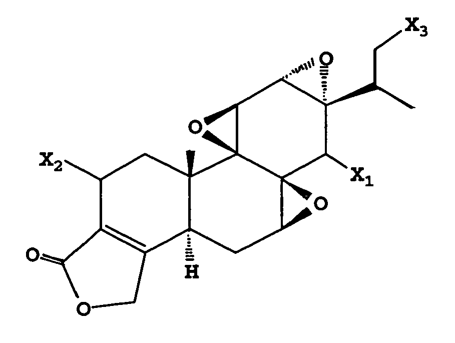 where X¹ is OH or OR¹, and X² and X³ are independently OH, OR¹ or H, with the proviso that at least one of X¹, X² and X³ is OR¹, and at least one of X² and X³ is H; and R¹ is -C(O)-Y-Z. In these embodiments, Y can be a branched or unbranched C₁-C₆ alkyl or alkenyl chain; and Z can be COOR², NR³R^{3'}, or +NR⁴R^{4'}R^{4"}, where R² is a cation; R³ and R^{3'} can be independently H or branched or unbranched C₁-C₆ alkyl, hydroxyalkyl, or alkoxyalkyl, or R³ and R^{3'} taken together can form a 5- to 7-member heterocyclic ring whose ring atoms can be selected from the group consisting of carbon, nitrogen, oxygen and sulfur. The ring atoms can include 2 to 6 carbon atoms and one or more nitrogen atoms, and optionally one or more oxygen or sulfur atoms, and the ring can be unsubstituted or substituted with other chemical moieties such as, for example, halogens. Each of the R⁴, R^{4'} , and R^{4"} can be independently branched or unbranched C₁-C₆ alkyl, hydroxyalkyl, or alkoxyalkyl groups. The triptolide prodrug, or a pharmaceutically acceptable salt thereof can be used for the manufacture of a medicament, optionally in a pharmaceutically acceptable vehicle, for the treatment of acute myeloid leukemia.

In some embodiments, the triptolide prodrug, or a pharmaceutically acceptable salt thereof, has the following structure: 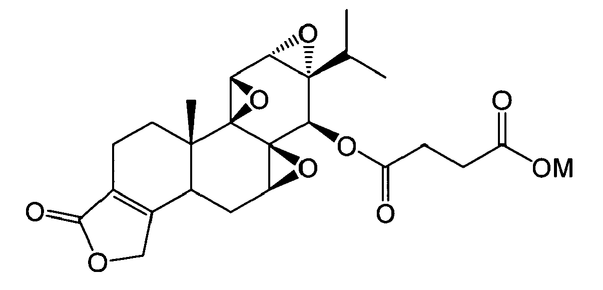 where M can be a H or a cation; particularly a pharmaceutically acceptable cation.

In many embodiments, the cation can be selected from a group consisting of a metal or an organic amine. In these embodiments, the metal can be Na or K, and the organic amine can be lysine, triethylamine, or tris(hydroxymethyl)aminomethane.

In some embodiments, the acute myeloid leukemia can be refractory or relapsed, and the treatments can result in complete remission with pancytopenia, complete remission without full platelets recovery, or complete remission.

### Synthesis of Triptolide Analogs

This section describes the synthesis of compounds in accordance with the present invention. In general, the compounds are ester derivatives of triptolide, tripdiolide or 16-hydroxytriptolide, wherein the attached ester substituents include one or more amino or carboxylate groups. The compounds possess greater water solubility than do the non-derivatized starting compounds and are useful as prodrugs for immunosuppressive and anti-inflammatory applications.

The compounds of the invention may be prepared from triptolide, tripdiolide, or 16-hydroxytriptolide obtained from the root xylem of the Chinese medicinal plant Tripterygium Wilfordii (TW) or from other known sources. The TW plant is found in the Fujiang Province and other southern provinces of China; TW plant material can generally be obtained in China or through commercial sources in the United States. Methods for preparing triptolide, tripdiolide, and 16-hydroxytriptolide are known in the art.

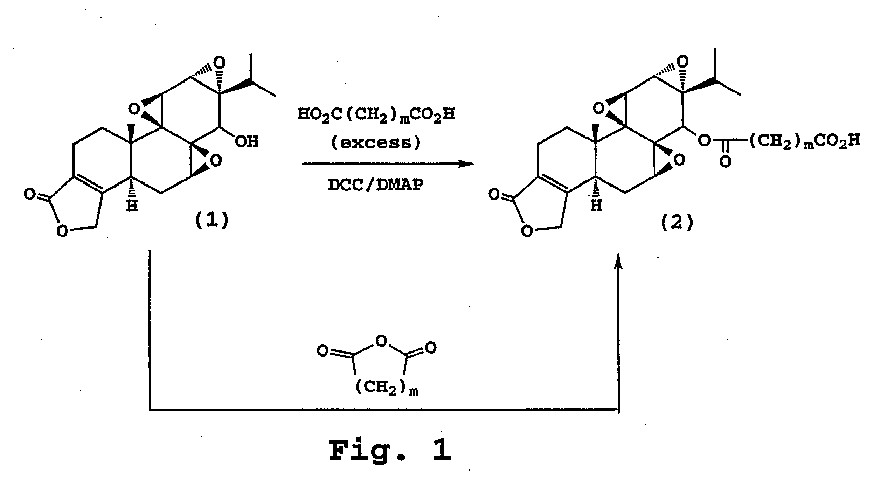

Synthetic schemes for preparing carboxylated derivatives of triptolide in accordance with the invention are shown in FIG. 1. With reference to the upper reaction path shown in the figure, triptolide (1) is reacted with an excess of a dicarboxylic acid of the form HO₂C(CH₂)ₘCO₂H, where m is, e.g. 1 to 4, in the presence of a coupling agent such as dicyclohexylcarbodiimide (DCC) and a catalytic amount of an acylation catalyst such as 4-(dimethylamino)pyridine (DMAP). The reaction conditions are effective to activate one or both carboxylate groups in the dicarboxylic acid towards reaction with the 14-hydroxyl group of (1), such that ester product (2) is formed. Any residual DCC attached to the free carboxyl in (2) may be released by addition of water, preferably under basic conditions.

A second method for preparing carboxylated derivatives of (1) is shown in the lower reaction path in FIG. 1. In this approach, (1) is reacted with a selected dicarboxylic acid anhydride, under conditions effective for the 14-hydroxyl group of (1) to attack one of the anhydride carbonyl groups to produce product (2). Exemplary conditions for this approach can be found in Example 1.

More generally, the methods illustrated in FIG. 1 can be used to prepare triptolide derivatives , wherein X² and X³ are H and X¹ is -C(O)-Y-Z (i.e., R¹), wherein Y is a branched or unbranched C₁-C₆ alkyl or alkenyl chain, and Z is COOR², where R² is a cation.

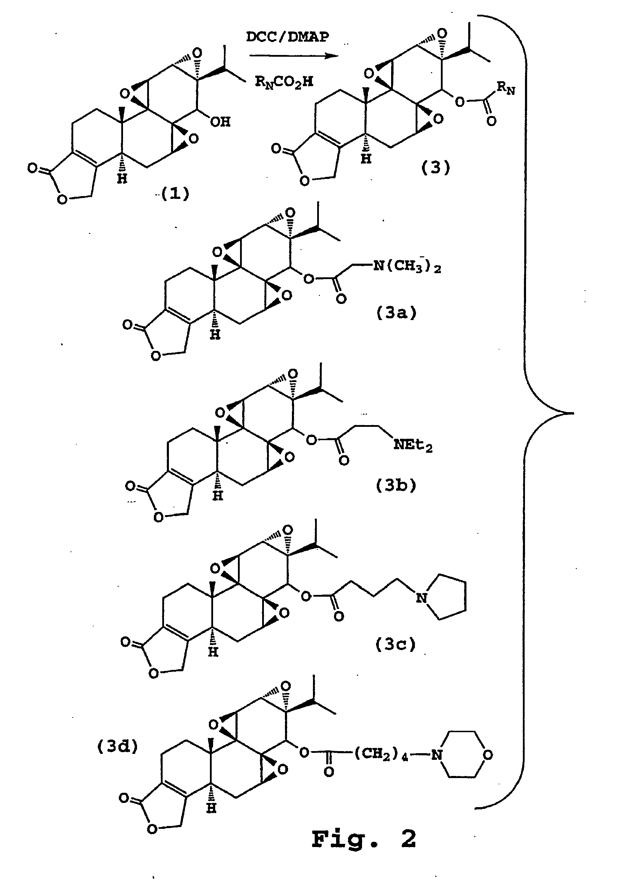

FIG. 2 illustrates a method for preparing amino derivatives of triptolide. Triptolide (1) is reacted with an amine-substituted carboxylic acid, R_{N}CO₂H in the presence of a coupling agent (e.g. DCC) and an acylation catalyst (e.g. DMAP). These reaction conditions may be used to prepare a number of amino derivatives, depending on the amino acid starting material. For example, reaction of (1) with N,N-dimethylglycine affords ester product (3a), as shown in FIG. 2 and described in Example 5. Similarly, reaction of (1) with 3-(N,N-diethylamino)propionic acid, 4-pyrrolidinobutyric acid, or 5-morpholinopentanoic acid affords product (3b), (3c), or (3d), respectively, as detailed in Examples 7-9. Amine salts in accordance with the invention are readily prepared by treatment with a selected acid, as in Example 6, or by using an ammonium salt form of R_{N}CO₂H in the coupling step, as in Examples 7 and 8.

Thus, it will be seen that the approach in FIG. 2 may be used to prepare amino derivatives in accordance with structure (3) in FIG. 2, wherein R_{N} has the form Y-Z as defined in the Summary of the invention, Y is a branched or unbranched C₁-C₆ alkyl or alkenyl chain, Z is NR³R^{3'} or ⁺NR⁴R^{4'}R^{4"}, R³ and R^{3'} are independently H or branched or unbranched C₁-C₆ alkyl, hydroxyalkyl, or alkoxyalkyl, or, taken together, form a 5- to 7-member heteroatom ring containing 2 to 6 carbon atoms, and R⁴, R^{4'}, and R^{4"} are independently branched or unbranched C₁-C₆ alkyl, hydroxyalkyl or alkoxyalkyl. In the case where Z is NR³R^{3'} where R³ and R^{3'} together form a ring and, in some embodiments, ring moieties include morpholine, piperidine, pyrrolidine, and piperazine.

Moreover, while FIGs. 1 and 2 illustrate reaction schemes using triptolide as the starting material, it will be appreciated that similar synthetic reaction schemes can be used to prepare corresponding ester derivatives of 16-hydroxytriptolide and tripdiolide.

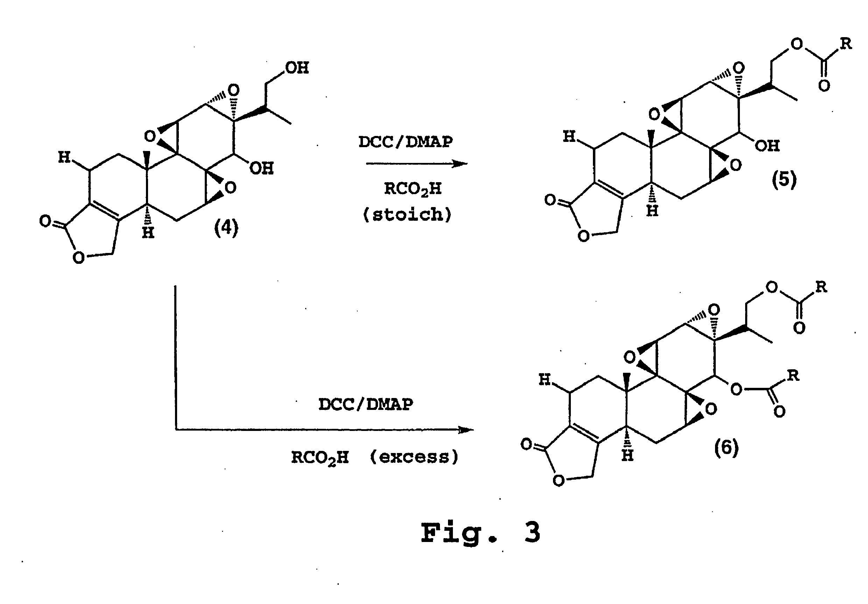

FIG. 3 illustrates synthetic approaches for preparing mono- and diester derivatives of 16-hydroxytriptolide (4), a compound which contains two free hydroxyl groups. As can be seen from the figure, compound (4) contains a hydroxyl group at the 14-position which is linked to a secondary carbon atom, and a second hydroxyl group at the 16-position which is linked to a primary carbon atom. Since the hydroxyl group at the 16-position is more reactive than the 14-hydroxyl group for steric reasons, mono- and diester derivatives can be selectively made using appropriate reaction conditions.

As shown in the upper reaction path of FIG. 3, reaction of (4) with a stoichiometric amount of a selected carboxylic acid yields monoester (5) derivatized at the 16-position, with the 14-hydroxyl group remaining free. Conversely, as shown in the lower reaction path, reaction of (4) with an excess of the carboxylic acid is effective to derivatize both hydroxyl groups, affording diester (6).

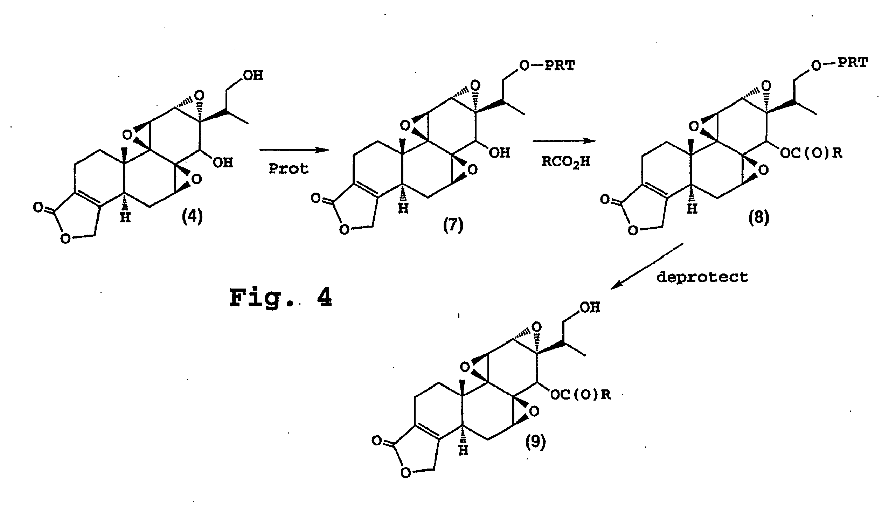

Monoester derivatives of 16-hydroxytriptolide (4) at the 14-position, rather than the 16-position, can be prepared by the general approach shown in FIG. 4. This approach takes advantage of the greater reactivity of the 16-hydroxyl group towards electrophiles, whereby the 16-hydroxyl can be selectively protected with a protecting group (PRT), as shown in the first step of FIG. 4. The protected compound (7) is then reacted with a selected carboxylic acid (RCO₂H) to esterify the 14-hydroxyl group, forming compound (8). The protecting group is then removed in a deprotection, to yield the desired 14-monoester (9). Suitable hydroxyl protecting groups for the purposes of the protection/deprotection scheme in FIG. 4 are known, and are described, for example, by Kocienski i, P.J. Protecting Groups, Georg Thieme Verlag, Stuttgart (1994). One preferred protecting group is a benzyl ether, which may be removed by catalytic hydrogenation (Kocienski, 1994, p. 46). Alternatively, a t-butyldimethyl silyl ether may be used. This group can be removed by, for example, treatment with tetrabutyl ammonium fluoride (TBAF).

Selective single derivatization of tripdiolide (2-hydroxytriptolide) is more difficult because of the similar reactivities of the two secondary hydroxyls. Accordingly, the 2- and 14-monoesters may be prepared as a mixture either by (1) reacting tripdiolide with a comparable amount of carboxylic acid (e.g., 1 to 3 equivalents) or (2) briefly reacting tripdiolide with excess carboxylic acid followed quickly by addition of excess alcohol (e.g., ethanol) to quench the excess carboxylic acid. In either case, a mixture of mono- and diester forms can be obtained which may then be separated by standard chromatographic methods such as HPLC.

Salts of the amino and carboxyl ester compounds for use in the invention are readily prepared by reaction or exchange with an appropriate counterion, as e.g. described in Examples 2-4 and 6. Suitable cations are defined above as pharmaceutically acceptable salts and inlcude metal ions and organic amines.

### Stability of Triptolide Derivatives

Sodium triptolide succinate was dissolved in D₂O, and the aqueous solution was stored at room temperature. Proton NMR spectra were taken at intervals and showed the compound to be unchanged after three months in solution. After five months, some decomposition was observed.

The stability of triptolide succinate in blood serum was determined. In this study, a solution of triptolide succinate (the free acid of the sodium triptolide succinate) in DMSO was mixed with rat serum and incubated at 37°C. The mixture was assayed periodically by thin layer chromatography (TLC) to follow the hydrolysis of the triptolide succinate over time. Within the first 3 to 5 minutes, most of the triptolide succinate remained (R_{f} =0.45). After 15 minutes, the triptolide succinate spot was gone and a new spot corresponding to triptolide had appeared (R_{f} =0.60). Finally, after 45 minutes, the triptolide spot had also disappeared, and only low R_{f} material (blood serum components and decomposition products) remained. These results indicate that the triptolide ester is hydrolyzed in serum to release free triptolide in less than an hour.

### Therapeutic Compositions

Formulations containing the active agents described above are not particularly restricted and may take the form of solid, semi-solid, lyophilized powder, or liquid dosage forms, such as tablets, capsules, powders, sustained-release formulations, solutions, suspensions, emulsions, ointments, lotions, or aerosols, preferably in unit dosage forms suitable for simple administration of precise dosages. The compositions typically include a conventional pharmaceutical carrier or excipient and may additionally include other medicinal agents, carriers, or adjuvants.

In some embodiments, the composition will be about 0.5% to 75% by weight of a compound or compounds of the invention, with the remainder consisting of suitable pharmaceutical excipients. For oral administration, such excipients can include pharmaceutical grades of mannitol, lactose, starch, magnesium stearate, sodium saccharine, talcum, cellulose, glucose, gelatin, sucrose, magnesium carbonate, and the like. If desired, the composition may also contain minor amounts of non-toxic auxiliary substances such as wetting agents, emulsifying agents, or buffers.

One of skill will appreciate that the dosage used can vary according to a variety of factors, such as age, sex, race, physical condition of the patient, presence of other diseases, use of other drugs, etc. In some embodiments, the treatment of acute myeloid leukemia can be performed by administering an ester derivatives of triptolide, tripdiolide, 16-hydroxytriptolide, or a combination thereof at a dose level that ranges from about 0.015 to about 30 mg/m²/day, from about 0.150 to about 13 mg/m²/day, from about 0.300 to about 10 mg/m²/day, from about 0.500 to about 8.5 mg/m²/day, from about 0.750 to about 8.0 mg/m²/day, from about 1.00 to about 7.5 mg/m²/day, from about 1.50 to about 7.0 mg/m²/day, from about 2.00 to about 6.5 mg/m²/day, from about 2.50 to about 6.0 mg/m²/day, from about 3.00 to about 5.5 mg/m²/day, or any range therein.

In some embodiments, the treatment of acute myeloid leukemia can be performed by administering a 14-succinyl triptolide sodium salt of triptolide at a dose level that ranges from about 5.2 mg/m²/day to about 6.2 mg/m²/day. In some embodiments, the dose level is about 5.7 mg/m²/day. In some embodiments, the dose level ranges from about 5.0 to about 9.0 mg/m²/day, from about 5.2 to about 8.7 mg/m²/day, from about 5.7 to about 8.5 mg/m²/day, or is about 7.0 mg/m²/day, or any range therein.

The composition may be administered to a subject orally, transdermally or parenterally, for example, by intravenous, subcutaneous, intraperitoneal, or intramuscular injection. For use in oral liquid preparation, the composition may be prepared as a solution, suspension, emulsion, or syrup, being supplied either in liquid form or a dried form suitable for hydration in water or normal saline. For parenteral administration, an injectable composition for parenteral administration will typically contain the triptolide analog in a suitable intravenous solution, such as sterile physiological salt solution.

Liquid compositions can be prepared by dissolving or dispersing the triptolide analog (about 0.5% to about 20%) and optional pharmaceutical adjuvants in a carrier, such as, for example, aqueous saline, aqueous dextrose, glycerol, or ethanol, to form a solution or suspension. The high water solubility of the compounds for use in the invention makes them particularly advantageous for administering in aqueous solution, for example by intraperitoneal injection. Although aqueous solutions are preferred, compositions in accordance with the invention may also be formulated as a suspension in a lipid (e.g., a triglyceride, a phospholipid, or a polyethoxylated castor oil such as CREMOPHOR EL), in a liposomal suspension, or in an aqueous emulsion.

Methods for preparing dosage forms are known or will be apparent to those skilled in the art; for example, see Remington's Pharmaceutical Sciences (19th Ed., Williams & Wilkins, 1995). The composition to be administered will contain a quantity of the selected compound in a pharmaceutically effective amount for effecting immunosuppression in a subject.

For systemic administration, the composition may be administered orally, transdermally or parenterally, for example, by intravenous, subcutaneous, intraperitoneal, or intramuscular injection, or by inhalation. Multiple intravenous, subcutaneous and/or intramuscular doses are possible, and in the case of implantable methods for treatment, formulations designed for sustained release are particularly useful. Patients may also be treated using implantable subcutaneous portals, reservoirs, or pumps.

For inhalation, the compound may be in the form of aerosol particles, either solid or liquid, preferably of respirable size. Such particles are sufficiently small to pass through the mouth and larynx upon inhalation and into the bronchi and alveoli of the lungs. In general, particles ranging from about 1 to 10 µm in size, and preferably less than about 5 µm in size, are respirable. Liquid compositions for inhalation comprise the active agent dispersed in an aqueous carrier, such as sterile pyrogen free saline solution or sterile pyrogen free water. If desired, the composition may be mixed with a propellant to assist in spraying the composition and forming an aerosol.

Regional treatment can be useful in some treatments and can be accomplished by intraarterial infusion. A catheter can be surgically or angiographically implanted to direct treatment to the affected organ. A subcutaneous portal, connected to the catheter, can be used for chronic treatment, or an implantable, refillable pump may also be employed.

The following examples illustrate, but do not limit, the present invention.

### EXAMPLES

### Example 1. Synthesis of Triptolide Succinate

Triptolide (100 mg) in 10 ml of pyridine can be treated with succinic anhydride (150 mg) at room temperature. The reaction can be carried out at 85°C for 30 hours under a nitrogen atmosphere. Hexane (50 ml) can be added to the resultant mixture to precipitate a crude product, collected by filtration and washed with hexane. The crude product can be recrystallized from ether/hexane to yield 90 mg (70%) of triptolide succinate, m.p. 111-113°C.
IR(KBr): 3431.8, 2974.6, 1743.8, 1375.5, 1159.4, 1022.4 cm⁻¹. ¹H NMR (CDCl₃): 5.08 (1 H, s, 14-CH), 4.67 (2H, s, 19-CH₂), 3.82 (1 H, d, 11-CH), 3.50 (1 H, d, 12-CH), 3.43 (1 H, d, 7-CH), 2.75 (5H, m, CH₂CH₂, 5-CH), 2.30 (1 H, d-m, 15-CH), 2.15 (2H, m, 6-CHₐ, 2-CHₐ), 1.88 (2H, m, 2-CH_{b}, 6-CH_{b} B), 1.55 (1 H, m, 1-CH_{b}), 1.20 (1 H, m, 1-CHₐ), 1.05 (3H, s, 20-CH₃), 0.95 (3H, d, 16-CH₃), 0.83 (3H, d, 17-CH₃) ppm. MS (m/z): 461 (M+1).

### Example 2. Synthesis of Triptolide Succinate Tris Salt

Triptolide succinate (20 mg) can be mixed with tris(hydroxymethyl)amino methane (5.3 mg) in 20 ml of water and stirred for one hour. The solution can be filtered and the filtrate lyophilized to yield 24 mg (96%) of white powder.
IR(KBr): 3391, 2937.96, 1745.80, 1562.9, 1411.67, 1159, 1066.57, 1024.57 cm⁻¹. ¹H NMR (D₆-DMSO, ppm): 5.00 (1H, s, 14-CH), 4.85 (2H, d, 19-CH₂), 3.95 (1 H, d, 11-CH), 3.70 (1 H, d, 12-CH), 3.55 (1 H, d, 7-CH), 3.30 (6H, s, 3CH₂O), 2.65 (1 H, m, 5H), 2.45 (2H, m, CH₂), 2.20 (3H, m, CH₂, 15-CH), 1.90 (4H, m, 6-CH₂, 2-CH₂), 1.34 (2H, br, 1-CH₂), 0.95 (3H, s, 20-CH₃), 0.88 (3H, d, 16-CH₃), 0.75 (3H, d, 17-CH₃).

### Example 3. Synthesis of Triptolide Succinate Sodium Salt

Triptolide succinate (20 mg) can be mixed with sodium bicarbonate (3.65 mg) in 20 ml of water and stirred for 30 min. The water solution can be filtered and the filtrate lyophilized to yield 20 mg (95%) white powder.
IR(KBr): 3431.8, 2975.56, 1743.87, 1577.97, 1419.79, 1163.22, 1022.40 cm⁻¹. ¹H NMR (D₆-DMSO, ppm): 5.00 (1 H, s, 14-CH), 4.85 (2H, d, 19-CH₂), 3.95 (1 H, d, 11-CH), 3.70 (1 H, d, 12-CH), 3.55 (1 H, d, 7-CH), 2.58 (1 H, m, 5H), 2.45 (2H, m, CH₂), 2.20 (3H, m, CH₂, 15-CH), 1.90 (4H, m, 6-CH₂, 2-CH₂), 1.34 (2H, br, 1-CH₂), 0.95 (-3H, s, 20-CH₃), 0.88 (3H, d, 16-CH₃), 0.75 (3H, d, 17-CH₃).

### Example 4. Synthesis of Triptolide Succinate Lysine Salt

Triptolide succinate (20 mg) can be mixed with L-(+)-lysine (6.3 mg) in 20 ml water and stirred for one hour. The solution can be filtered and the filtrate lyophilized to yield 25 mg (95%) white powder.
IR(KBr): 3431.8, 2934.0, 1743.9, 1560.6, 1399.9, 1147.6, 1018.6 cm⁻¹. ¹H NMR (D₆ -DMSO, ppm): 5.00 (1 H, s, 14-CH), 4.85 (2H, d, 19-CH₂), 3.95 (1 H, d, 11-CH), 3.78 (1 H, d, 12-CH), 3.55 (1 H, d, 7-CH), 3.50 (6H, br, 2NH₃), 3.15 (1 H, m, - CH), 2.70 (1 H, m, 5H), 2.65 (1 H, m, CH₂), 2.4 (2H, m, CH₂), 2.20 (3H, m, CH₂, 15-CH), 1.90 (4H, m, 6-CH₂, 2-CH₂), 1.40 (6H, m, CH₂CH₂CH₂), 1.34 (2H, br, 1-CH₂), 0.95 (3H, s, 20-CH₃), 0.88 (3H, d, 16-CH₃), 0.75 (3H, d, 17-CH₃) ppm.

### Example 5. Synthesis of 14-N,N-Dimethylglycinate Ester of Triptolide

Into a dry 100 mL round bottom flask can be placed 1 eq. of triptolide and 2 eq. each of N,N-dimethyl glycine and DCC (dicyclohexylcarbodiimide). The flask can be placed under a nitrogen atmosphere, and anhydrous CH₂Cl₂ (dried over P₂O₅) added, followed by a catalytic amount of DMAP (4-dimethylaminopyridine). The solution can be stirred overnight at room temperature. The reaction can be worked up by filtering off the dicyclohexylurea, removing the solvent by evaporation, and chromatographing the obtained solid on silica gel.

### Example 6. Synthesis of Methanesulfonic Acid Salt of 14-N,N-Dimethylglycinate Ester of Triptolide

Into a dry round bottom flask can be placed 1 eq. of the 14-N,N-dimethylglycinate ester of triptolide, as prepared in Example 5. The compound can be dissolved in anhydrous CH₂Cl₂ (distilled from P₂ O₅), and to the resulting solution added 1 eq. of a stock solution of methanesulfonic acid in diethyl ether. The solvent can be immediately removed to yield a white solid.

### Example 7. Synthesis of 14-(3-(N,N-Dimethylamino)propionate) Hydrochloric Salt Ester of Triptolide

Into a dry 100 mL round bottom flask can be placed 1 eq. of triptolide and 2 eq. each of N,N-dimethylamino propionic acid and DCC (dicyclohexylcarbodiimide). The flask can be placed under a nitrogen atmosphere, and anhydrous CH₂Cl₂ (dried over P₂O₅) added, followed by a catalytic amount of DMAP (4-dimethylaminopyridine). The solution can be stirred overnight at room temperature. The dicyclohexylurea can be filtered off, and the solvent removed by evaporation. The crude product can then be chromatographed on silica gel.

### Example 8. Synthesis of 14-(4'-N-pyrrolidino)butyrate) Hydrochloride Salt Ester of Triptolide

Into a dry round bottom flask can be placed 1 eq. of triptolide, 2 eq. of 4-pyrrolidinobutyric acid hydrochloride salt, and anhydrous CH₂Cl₂ (distilled from P₂O₅). The resulting solution can be placed under a nitrogen atmosphere, and 2 eq. of DCC and a catalytic amount of DMAP added. The solution can be stirred overnight at room temperature. The reaction can be worked up by filtering off the dicyclohexylurea, removing the solvent by evaporation, and chromatographing the obtained solid on silica gel.

### Example 9. Synthesis of Bis-N,N-Dimethylglycinate Ester of 16-Hydroxytriptolide

The compound can be synthesized by the reaction of 1 eq. of 16-hydroxytriptolide, 3 eq. of N,N-dimethylglycine, 3.3 eq. of DCC, and 0.16 eq. of DMAP in anhydrous CH₂Cl₂, followed by working up as described in the previous example.

The bis-N,N-dimethylglycinate ester at the 2- and 14-positions of triptolide can be prepared in a similar fashion from tripdiolide (2-hydroxytriptolide).

### Example 10. Stability of Triptolide Succinate

Stability in Water: a solution of sodium triptolide succinate in D₂O can be prepared at a concentration of 3 mg/ml and stored at room temperature. The solution can be analyzed by ¹H NMR at intervals of 1, 3, 5, 15, 45, 90, 180 minutes; 1, 7, 14 days; and 1, 2, 3, and 5 months. No appreciable change in the NMR spectrum should be seen during the first three months. Some decomposition may be apparent after 5 months.

Stability in Blood Serum: a solution of triptolide succinate (free acid) in DMSO can be made at a concentration of 25 mg/ml, and 0.1 ml of this solution can be mixed with 0.5 ml of rat serum. The mixture was incubated at 37°C. Aliquots of the mixture should then be taken at 1, 3, 5, 15, 45 minutes and 18 hours and analyzed by thin layer chromatography (TLC). The TLC plates can be developed in 1:5 CH₂Cl₂/Et₂O. After development, the plates can be treated with iodine vapor and examined under a UV lamp. Triptolide and triptolide succinate can be used as reference compounds (R_{f} =0.60, and 0.45, respectively).

After 3 minutes, only triptolide succinate may be detected by TLC. After 15 minutes, the triptolide succinate spot may be gone (R_{f} =0.45), and a new spot corresponding to triptolide may have appeared (R_{f} =0.60). After 45 minutes, the triptolide spot may also disappear, and only low R_{f} material (blood serum components and decomposition products) may remain.

### Example 11. Treatment of Acute Myeloid Leukemia using PG 490-88

PG490-88 Na is a 14-succinyl triptolide sodium salt of triptolide (PG490). In order to prove that PG490-88 Na can be used in the manufacture of a drug for treating acute myeloid leukemia (AML), the following experiment was designed and performed.

In this example, 29 patients with relapsed or refractory leukemias were treated. Median age was 57 years (range : 30 - 79). Sixty two percent of patients were male. Twelve dose levels from 0.150 to 13 mg/m²/day were tested in one patient each, with an increase by 50% for each successive cohort of patients until one dose limiting toxicity (DLT) was observed. Thereafter, dose escalation was switched to a standard design using cohorts of 3 to 6 patients per dose level, with 30% dose increments.

Among all the patients, 6 (24%) presented with a disease refractory to standard induction chemotherapy and the others were in 1^{st} or 2^{nd} relapse who had failed salvage therapy for 9 of them. Table 1 shows the experimental design.

**Table 1.**

| Dose level | PG490-88 Na | No. of patients |
|---|---|---|
| | (mg/m²/day) | |
| 1 | 0.150 | 1 |
| 2 | 0.225 | 1 |
| 3 | 0.335 | 1 |
| 4 | 0.500 | 1 |
| 5 | 0.750 | 1 |
| 6 | 1.125 | 1 |
| 7 | 1.7 | 1 |
| 8 | 2.5 | 1 |
| 9 | 3.8 | 1 |
| 10 | 5.7 | 6 |
| 1* | 7 | 7 |
| 11 | 8.5 | 6 |
| 12 | 13.0 | 1 |
| Total | | 29 |

| | | |
|---|---|---|
| * intermediate dose level as per protocol | | |

The maximum tolerated dose (MTD) was reached at 7 mg/m²/day and the recommended dose for this regimen was 5.7 mg/m²/day. At the recommended dose of 5.7 mg/m²/day, the only related adverse events were grade 3 leucopenia reported for 1 patient, and mild diarrhoea, nausea and vomiting. At doses > 5.7 mg/m²/day, the most prevalent toxicity was cerebellar toxicity which occurred in 5 patients : 3 patients (one grade 2 and two grade 4) at 7 mg/m²/day and 2 patients (two grade 4) at 8.5 mg/m²/day. Cerebellar toxicity happened between 13 and 17 days after the first study drug administration, and the toxicity generally consisted of ataxia, dysmetria and dysarthria.

One patient treated at 5.7 mg/m²/day reached the definition of "complete remission (CR) with pancytopenia" as defined by the protocol on day 15 of the treatment (i.e. total clearance of bone marrow blasts with secondary leukemia regrowth). This patient received only one cycle of treatment and could not receive additional cycles due to life threatening infectious complications.

Another patient treated at 8.5 mg/m²/day met the definition of "CRp: complete remission without full platelets recovery" (i.e. normalisation of the blood and the bone marrow with ≤5% blast cells; normocellular or hypocellular bone marrow; granulocyte count >1.5 x 10⁹/l, platelet transfusion independence but with platelet count remaining below 100 x 10⁹/l), after 2 cycles of treatment. The patient presented with a grade 4 aplasia lasting 20 days after the first cycle, and had no haematological toxicity after cycle 2.

A third patient treated also at 8.5 mg/m²/day reached complete remission (i.e. normalisation of the blood and bone marrow with ≤5% blast cells; normocellular or hypocellular bone marrow; granulocyte count >1.5 x 10⁹/l and platelet count >100 x 10⁹/l, lasting for at least 4 weeks) after 2 cycles of treatment. This patient presented with a moderate leucopenia (grade 1 lasting 2 days) after the first cycle and had no haematotoxicity during the second cycle, with a progressive normalisation of the blood count.

These results, obtained through exploratory dose escalation experiments, show a very good response from PG490-88Na on relapsed or refractory acute myeloid leukemia, making PG490-88 Na a valuable molecule for acute myeloid leukemia treatment.

The described embodiments are considered in all respects as illustrative and not restrictive. It should also be understood that the invention is not limited to the particular embodiments described herein, but is capable of many equivalents, rearrangements, modifications, and substitutions without departing from the scope of the invention.

## Claims

1. Use of a triptolide prodrug, or a pharmaceutically acceptable salt thereof, having the following structure: 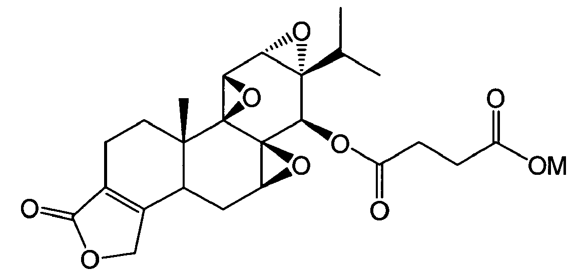 where M is a H or a cation;
for the manufacture of a medicament comprising the triptolide prodrug or a pharmaceutically acceptable salt thereof for the treatment of acute myeloid leukemia.

2. The use of claim 1, wherein the cation is selected from the group consisting of a metal or an organic amine.

3. The use of claim 2, wherein the metal is Na or K.

4. The use of claim 2, wherein the metal is Na.

5. The use of claim 2, wherein the organic amine is lysine, triethylamine, or tris(hydroxymethyl) aminomethane.

6. The use of any of claims 1 to 5, wherein the triptolide prodrug is to be administered in an amount ranging from 5.0 mg/m²/day to 9.0 mg/m²/day.

7. The use of claim 6, wherein the triptolide prodrug is to be administered in an amount ranging from 5.2 mg/m²/day to 8.7 mg/m²/day.

8. The use of claim 7, wherein the triptolide prodrug is to be administered in an amount ranging from 5.7 mg/m²/day to 8.5 mg/m²/day.

9. The use of claim 8, wherein the triptolide prodrug is to be administered at an amount of about 7.0 mg/m²/day.

10. The use of any of claims 1 to 9, wherein the acute myeloid leukemia is refractory.

11. The use of any of claims 1 to 9, wherein the acute myeloid leukemia is relapsed.

12. The use of claims 1 to 11, wherein the treatment results in complete remission with pancytopenia.

13. The use of claims 1 to 11, wherein the treatment results in complete remission without full platelets recovery.

14. The use of claims 1 to 11, wherein the treatment results in complete remission.
